(19) Europäisches Patentamt
European Patent Office
Office européen des brevets

(11) Veröffentlichungsnummer: **0 028 829**
**B1**

# (12) EUROPÄISCHE PATENTSCHRIFT

(45) Veröffentlichungstag der Patentschrift: **28.09.83**

(21) Anmeldenummer: **80106895.8**

(22) Anmeldetag: **08.11.80**

(51) Int. Cl.³: **C 07 C 127/15**, C 07 C 127/19, C 07 C 127/22, C 07 C 157/05, C 07 C 157/09, C 07 D 211/58, A 01 N 47/28

## (54) Harnstoffe mit cyclischen Substituenten, ihre Herstellung und Verwendung als Herbizide.

(30) Priorität: **10.11.79 DE 2945530**

(43) Veröffentlichungstag der Anmeldung: **20.05.81 Patentblatt 81/20**

(45) Bekanntmachung des Hinweises auf die Patenterteilung: **28.09.83 Patentblatt 83/39**

(84) Benannte Vertragsstaaten: **BE CH DE FR GB IT LI LU NL SE**

(56) Entgegenhaltungen:
**AT-B-261 304**
**DE-A-1 567 010**
**US-A-3 534 141**

(73) Patentinhaber: **CHEMISCHE WERKE HÜLS AG, Postfach 1320, D-4370 Marl 1 (DE)**

(72) Erfinder: **Samblebe, Reinhard, Dr., An der Ziegelei 48, D-4690 Herne 2 (DE)**
Erfinder: **Baltruschat, Helmut, Dr., Schwalbenweg 2, D-4408 Dülmen (DE)**
Erfinder: **Schnurbusch, Horst, Dr., Overwegstrasse 36, D-4690 Herne 1 (DE)**
Erfinder: **Hasser, Günter, Dr., Zillenbachstrasse 50, D-8973 Hindelang (DE)**

(74) Vertreter: **Steil, Hanna, Dipl.-Chem., RSP PATENTE - PB 15 Postfach 1320, D-4370 Marl 1 (DE)**

**EP 0 028 829 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Harnstoffe mit cyclischen Substituenten, ihre Herstellung und Verwendung als Herbizide

Cycloalkylderivate des Harnstoffs und Thioharnstoffs der allgemeinen Formel

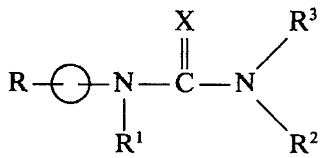

mit Ringen aus 5 – 8 C-Atomen, in der R, $R^1$, $R^2$ und/oder $R^3$ Wasserstoff oder eine Alkylgruppe oder $R^1$, $R^2$ und/oder $R^3$ eine Cycloalkyl-, oder Alkenyl- oder Arylgruppe bedeuten und X jeweils Sauerstoff oder Schwefel und $R^2$ und $R^3$ auch Teile eines gemeinsamen Ringes sein können, sind bekannt und als Verbindungen mit herbiziden Wirksamkeiten erkannt worden.

Bekannt sind auch Verbindungen entsprechend aufgebauter Harnstoffe, die bis zu drei Methylgruppen in untereinander nicht benachbarten Positionen an einem Cyclohexylring tragen können.

So ist z. B. die Herstellung von Harnstoffen nach allgemein bekannten Verfahren aus Dimethylcyclohexylaminen (2,6-, 2,4-, 3,5-, 2,5-Dimethylcyclohexylamine) in US-PS 3 347 658 genannt. Für die Darstellung von N,N' symmetrisch aliphatisch disubstituierten Allophanylchloriden — die ihrerseits durch thermische Zersetzung unter Abspaltung von Chlorwasserstoff die entsprechenden Isocyanate ergeben — wird N,N'-Di(2,4,6-trimethylcyclohexyl)-harnstoff verwendet, siehe US-PS 3 275 669 und 3 337 621.

Die Wirksamkeiten der vorgenannten Cycloalkylharnstoffe sind jedoch sowohl als Total- als auch als Vorauflaufherbizide und für wichtige Kulturpflanzen auch im Nachauflauf immer noch unbefriedigend, weil ihre herbizide Wirkung zu gering und gegen bestimmte weit verbreitete Schadgräser nicht selektiv genug ist, da mit höheren Aufwandmengen ihre Phytotoxizität schnell zunimmt.

Die DE-OS 1 567 010 betrifft Cycloalkylphenylharnstoffe als Herbizide, wobei der Cycloalkylring allenfalls 2 Methylsubstituenten — und diese nicht in geminaler Stellung — trägt. Ähnliches gilt für die Harnstoffe gemäß OE-PS 261 304. Sie besitzen allerdings nur einen Ring, und zwar entweder einen Phenyl- oder einen Cycloalkylring. Schließlich zeigt die US-PS 3 534 141 bicyclisch substituierte Harnstoffe mit insektizider Wirkung.

Gemäß den US-PS 3 671 637 und 3 686 303 sind Verbindungen der allgemeinen Struktur

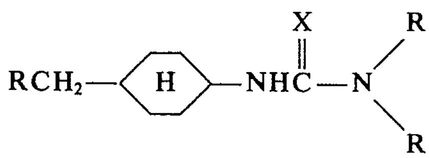

beschrieben worden, die als Repellents geeignet sind. Es wurde nun überraschend gefunden, daß bestimmte, bisher nicht bekannte dreifach und mehrfach substituierte Cycloalkylharnstoffe, insbesondere solche, die auch geminale Substituenten am Cycloalkylgerüst tragen, ferner auch Harnstoffe mit heterocyclischen Ringgliedern, die in der vorgenannten Weise substituiert sind, gegenüber bekannten vergleichbaren Harnstoffderivaten eine deutlich verbesserte herbizide Wirkung zeigen.

Gegenstand der vorliegenden Erfindung sind Harnstoffe mit cyclischen Substituenten der folgenden allgemeinen Formel I

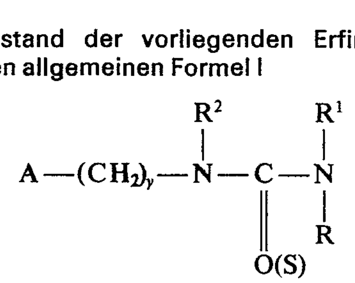

(I)

in der A =

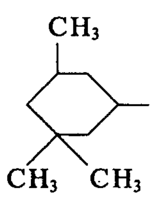

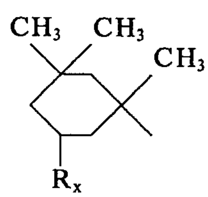

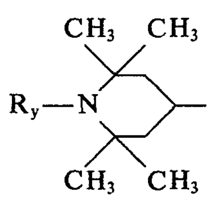

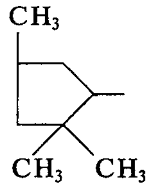

und

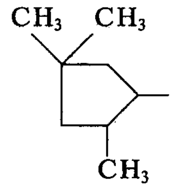

einschließlich der Stellungsisomeren, insbesondere der trans-Form mit

$R_x$ = OH, Carboxyl;
$R_y$ = H, Acyl
R = Wasserstoff, Hydroxyl, Alkyoxyl oder Acyl, einen $C_1-C_{12}$-Alkylrest gegebenenfalls mit Hydroxylgruppen, der geradkettig oder verzweigt und durch Sauerstoff und/oder Stickstoff-Atome unterbrochen sein kann, einen $C_3-C_{12}$-Cycloalkylrest gegebenenfalls mit Alkylsubstituenten, die durch Sauerstoff- und/oder Stickstoff-Atome unterbrochen sein können, und/oder mit Hydroxyl- und/oder mit einem aliphatischen oder aromatischen Acyl- und/oder mit Carboxyl- oder Carbalkoxygruppen, einen $C_2-C_5$-Alkenyl- oder Alkinylrest, einen Phenylrest, der durch Alkyl- und/oder Acyl- und/oder Hydroxyl- und/oder Dialkylaminogruppen substituiert sein kann;
$R^1$ = Wasserstoff oder einen geradkettigen oder verzweigten $C_1-C_4$-Alkylrest oder Alkoxyrest;
$R^2$ = Wasserstoff oder einen geradkettigen oder verzweigten $C_1-C_4$-Alkylrest gegebenenfalls mit Hydroxylgruppen, oder $C_1-C_4$-Acylrest und
$\gamma$ = eine Zahl von 0 bis 1

bedeuten.

Unter den neuen Verbindungen gemäß der allgemeinen Formel sind solche bevorzugt, in denen von den Resten R, $R^1$ und $R^2$ ein Rest aus Wasserstoff oder Acyl oder Alkyl besteht. Der Rest A weist stets wenigstens eine geminale Substitution am Ringsystem auf.

Im Falle von $\gamma=0$ sind unter anderem solche Verbindungen der angegebenen Formel besonders bevorzugt, in denen $R^2$ Wasserstoff oder einen Acylrest bedeutet.

Die neuen cyclischen Harnstoffe gemäß der vorstehenden Formelbeschreibung besitzen deutlich bessere herbizide Eigenschaften als die bisher verwendeten Harnstoffderivate. Insbesondere dann, wenn eine Vorauflauf- oder Nachauflaufbehandlung durchgeführt wird, weisen diese Verbindungen eine herausragende unkrautbekämpfende Wirkung auf, wobei sie gegenüber Kulturpflanzen nicht phytotoxisch sind.

Weiterer Gegenstand der Erfindung ist daher auch die Verwendung der Harnstoffe mit cyclischen Substituenten nach Anspruch 1 als Herbizide.

Die Darstellung der erfindungsgemäßen Verbindungen der allgemeinen Formel I kann nach verschiedenen, für analoge Verbindungen bekannten Methoden erfolgen:

Man erhält die neuen Verbindungen der Formel I zum Beispiel:

1a) durch Umsetzungen von Verbindungen der allgemeinen Formel III mit Verbindungen der allgemeinen Formel II:

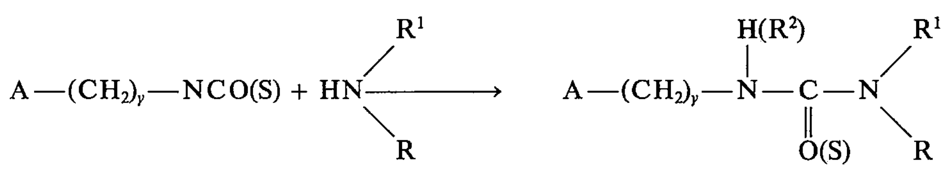

II III I

in diesem Falle haben

$\gamma$, R, $R^1$ und A die in der Formel I angegebene Bedeutung, jedoch darf A keine Hydroxylgruppe beinhalten,

$R^2$ ist bei diesen Umsetzungen immer Wasserstoff oder

1b) durch Umsetzungen von Verbindungen der allgemeinen Formel IV mit Verbindungen der allgemeinen Formel V:

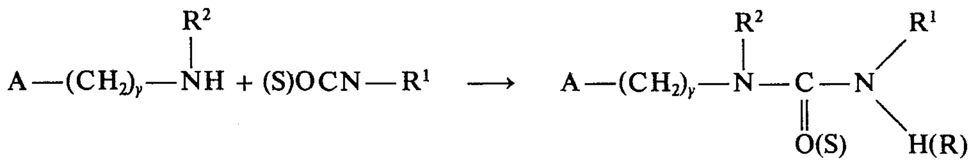

IV V I

in diesem Falle haben

$\gamma$, $R^1$, $R^2$ und A die in der Formel I angegebene Bedeutung, d. h. gegebenenfalls kann hier auch A eine Hydroxylgruppe enthalten.

R ist bei diesen Umsetzungen immer Wasserstoff.

Die Ausgangsstoffe der Formeln II bis V sind nur zum Teil literaturbekannt bzw. sie werden nach an sich bekannten Methoden hergestellt.

Bei der Umsetzung zu den neuen Harnstoffen gemäß der Formel I arbeitet man in inerten Lösungsmitteln wie Aceton, Äther, Dioxan, Benzin, Benzol, Toluol, Xylol und chlorierten aliphatischen oder aromatischen Kohlenwasserstoffen bei Temperaturen von 0°C bis zum Siedepunkt des Gemisches, vorzugsweise bei Temperaturen zwischen 20° und 160°C gegebenenfalls unter Zusatz von tertiären Alkylaminen oder Metallsalzen wie z. B. vorzugsweise Dibutylzinndilaurat (DBTL), Aluminium- oder Zink-alkyl/alkoxyl und/oder -halogenverbindungen.

Falls man eine nach den Formeln III und IV hydroxyl- oder auch carboxylhaltige Komponente für diese Reaktion einsetzt, so tropft man am besten das in einem inerten Lösungsmittel gelöste Isocyanat unter ständigem Rühren in eine vorgelegte Lösung des Amins.

Man erhält die neuen Verbindungen der Formel I ferner zum Beispiel:

2a) durch Umsetzung von Carbamoylchloriden der allgemeinen Formel VI mit primären oder sekundären Aminen der Formel VII in äquivalenter oder überschüssiger Menge unter basischen Bedingungen:

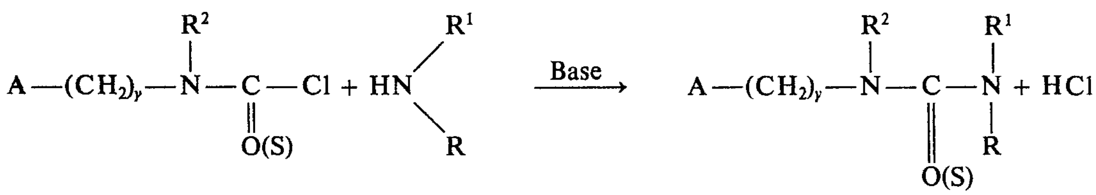


VI VII I

oder

2b) durch Umsetzung vom Carbamoylchloriden der allgemeinen Formel VIII mit primären oder sekundären Aminen der Formel IX in äquivalenter oder überschüssiger Menge unter basischen Bedingungen

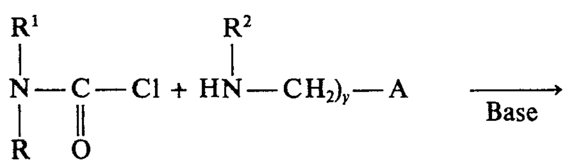


VIII IX

Im Falle des Herstellungsweges nach 2a) haben $\gamma$, R, $R^1$, $R^2$ und A die in der Formel I angegebene Bedeutung, jedoch darf $R^2$ nicht Wasserstoff, Hydroxyl, hydroxylhaltiger Alkylsubstituent oder Acyl sein und A darf auch kein Hydroxyl beinhalten. Im Falle des Herstellungsweges nach b) haben $\gamma$, R, $R^1$, $R^2$ und A die in der Formel I angegebene Bedeutung, jedoch darf hier weder R noch $R^1$ Wasserstoff, Hydroxyl oder Acyl sein oder einen hydroxylhaltigen aliphatischen Substituenten besitzen.

Die Ausgangsstoffe der Formeln VI bis IX sind zum Teil literaturbekannt bzw. werden nach an sich bekannten Methoden hergestellt. Bei ihrer Umsetzung zu den neuen Harnstoffen entsprechend der Form I arbeitet man in inerten Lösungsmitteln wie Ketonen, Äther, Dioxan, Benzin, Benzol, Toluol, Xylol und chlorierten aliphatischen oder aromatischen Kohlenwasserstoffen bei Temperaturen von 0°C bis zum Siedepunkt des Gemisches vorzugsweise bei Temperaturen zwischen 70°C und 220°C in Gegenwart von HCl-bindenden Basen, z. B. tertiären Aminen wie Pyridin oder Triäthylamin oder

Pottasche im wasserfreien Medium. Nach Beendigung der Reaktion wird das gebildete Salz mit Wasser, anschließend nochmals mit wäßriger alkalischer Lösung bis zur Neutralreaktion ausgewaschen, die Lösung getrocknet und eingeengt, dabei fällt oft der gebildete Harnstoff bereits in festen Kristallen aus.

Spezielle Vertreter der so erhaltenen Verbindungen können durch weitere bekannte Reaktionen in andere Verbindungen der allgemeinen Formel I überführt werden, so z. B. durch Verseifung, Salzbildung, Veresterung, Umesterung, Alkylierung, Acylierung oder Alkylolierung. In den meisten Fällen ist es jedoch vorteilhafter, die für die Harnstoffbildung benötigten Komponenten für die Herstellungswege 1a) und b) sowie 2a) und b) vorher bereits entsprechend der gewünschten Verbindung der allgemeinen Formel I aufzubauen.

Einige typische Beispiele an Verbindungen gemäß der Erfindung, die der angegebenen Formel I entsprechen, sind in der folgenden Tabelle I angegeben:

Tabelle 1

| Beispiel Nr. | Chemische Struktur |
|---|---|
| 1 | 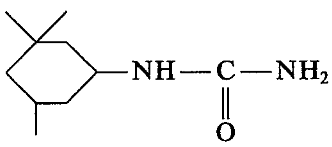 |
| 2 | 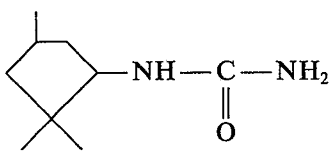 |
| 3 | 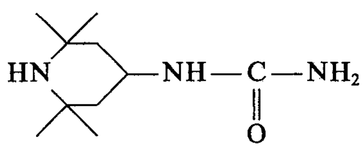 |
| 4 | 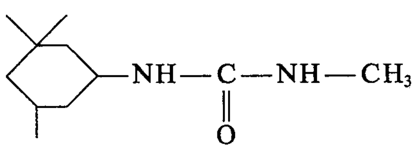 |
| 5 | 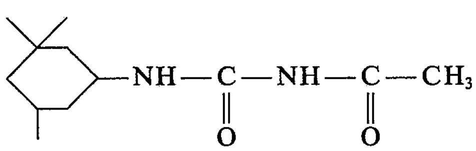 |
| 6 | 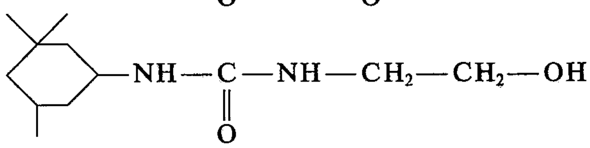 |
| 7 | 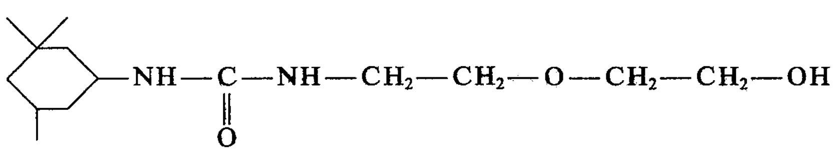 |
| 8 | 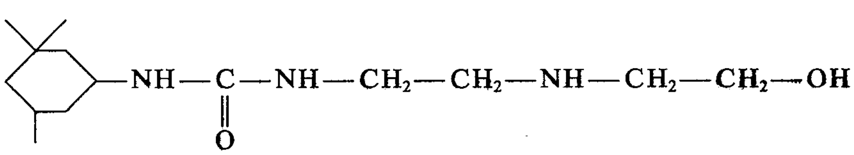 |

Fortsetzung

| Beispiel Nr. | Chemische Struktur |
|---|---|
| 9 | 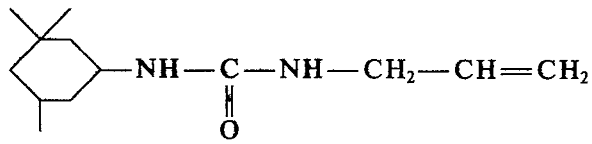 |
| 10 | 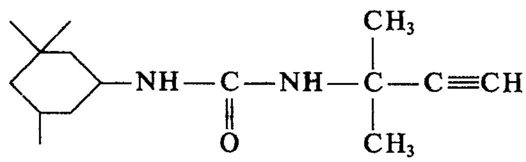 |
| 11 | 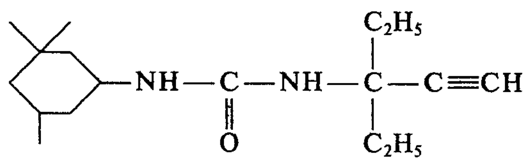 |
| 12 | 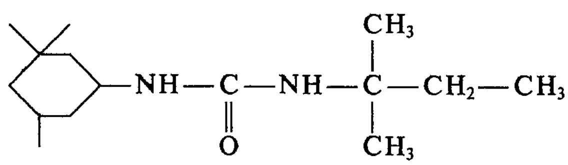 |
| 13 | 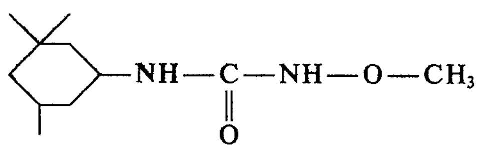 |
| 14 | 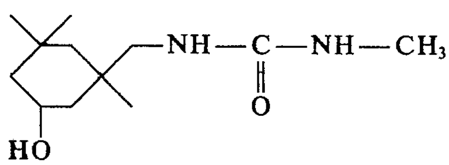 |
| 15 | 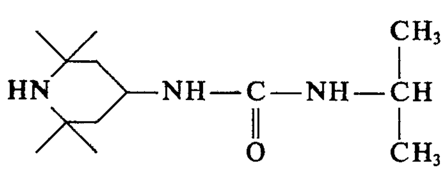 |
| 16 | 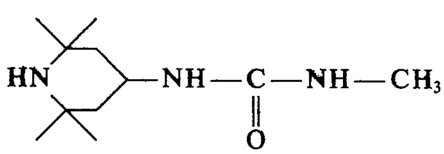 |
| 17 | 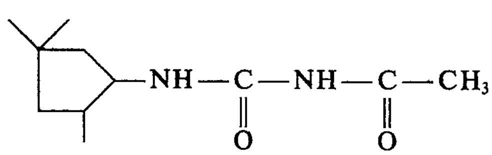 |

Fortsetzung

| Beispiel Nr. | Chemische Struktur |
|---|---|
| 18 | 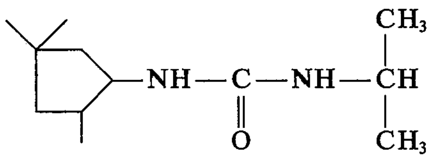 |
| 19 | 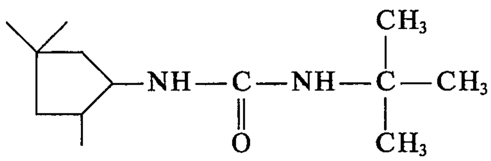 |
| 20 | 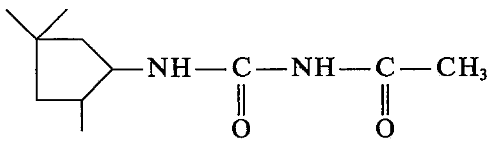 |
| 21 | 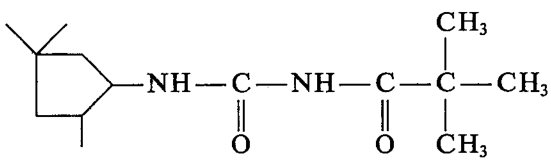 |
| 22 | 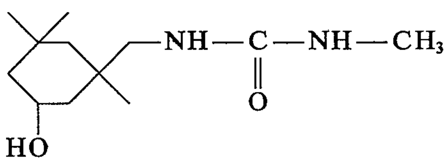 |
| 23 | 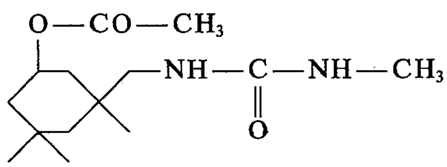 |
| 24 | 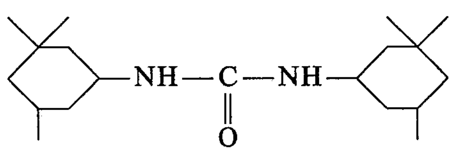 |
| 25 | 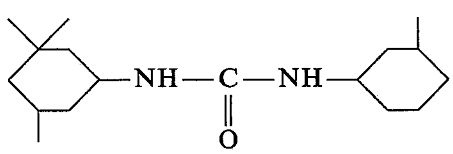 |
| 26 | 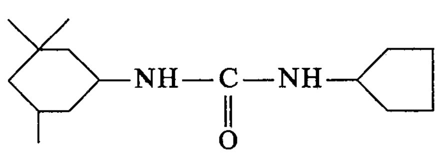 |

Fortsetzung

| Beispiel Nr. | Chemische Struktur |
|---|---|
| 27 | 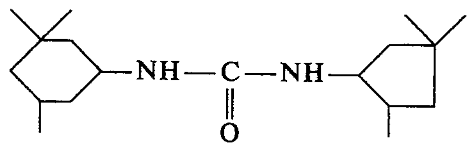 |
| 28 | 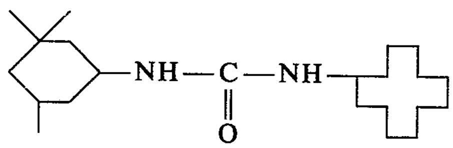 |
| 29 | 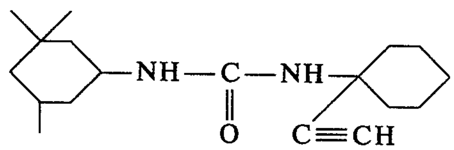 |
| 30 | 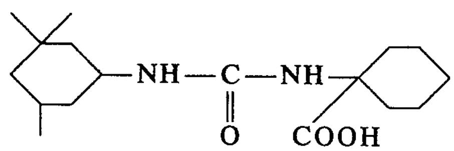 |
| 31 | 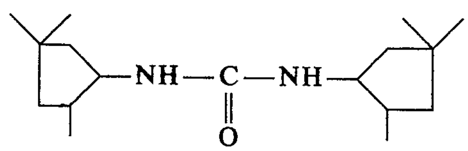 |
| 32 | 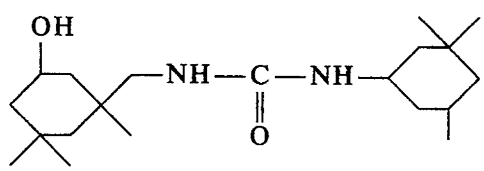 |
| 33 | 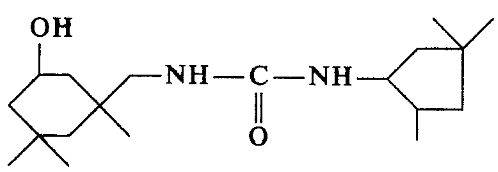 |
| 34 | 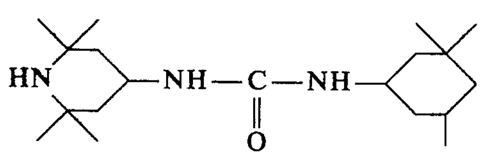 |
| 35 | 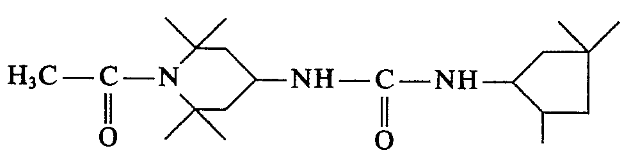 |
| 36 | 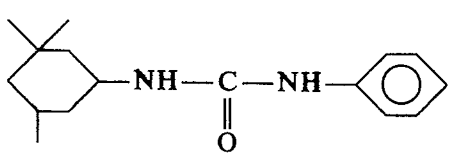 |

Fortsetzung

| Beispiel Nr. | Chemische Struktur |
|---|---|
| 37 | 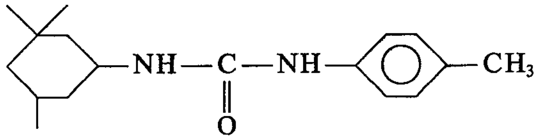 |
| 38 | 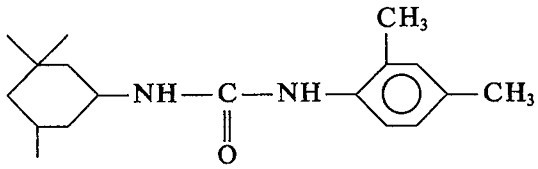 |
| 39 | 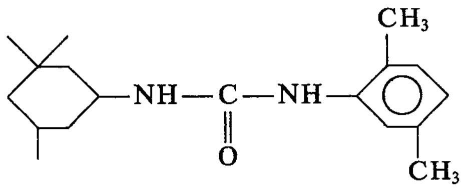 |
| 40 | 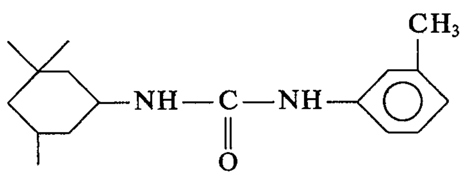 |
| 41 | 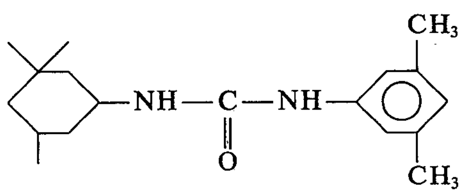 |
| 42 | 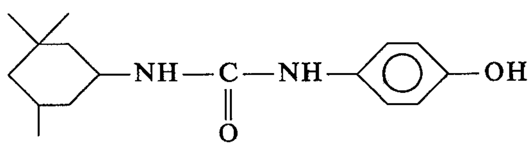 |
| 43 | 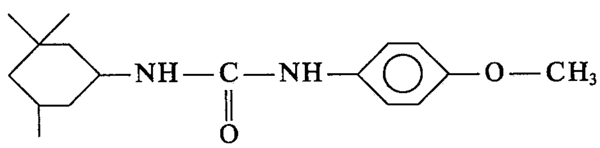 |
| 44 | 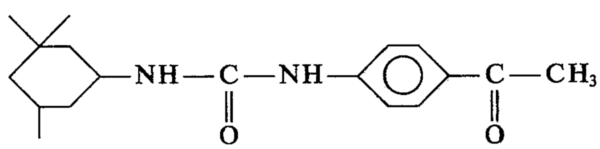 |
| 45 | 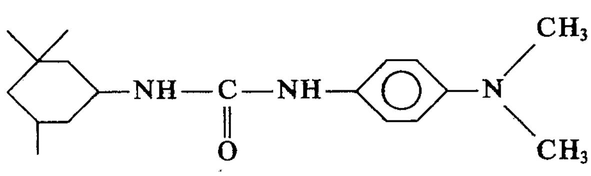 |
| 46 | 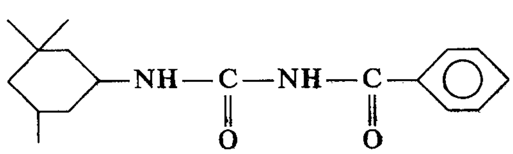 |

Fortsetzung

| Beispiel Nr. | Chemische Struktur |
| --- | --- |
| 47 | 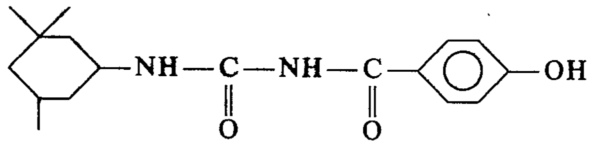 |
| 48 | 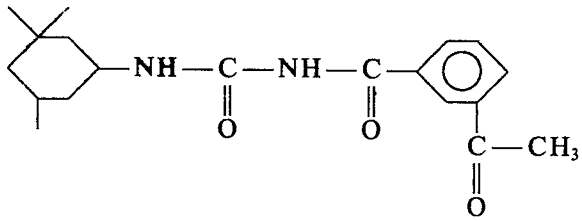 |
| 49 | 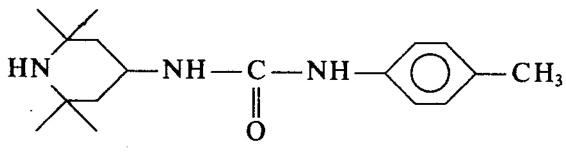 |
| 50 | 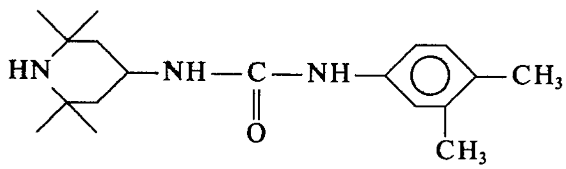 |
| 51 | 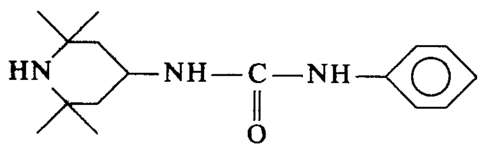 |
| 52 | 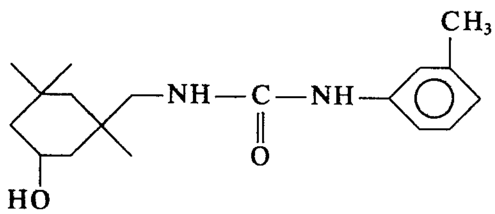 |
| 53 | 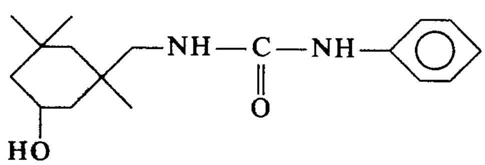 |
| 54 | 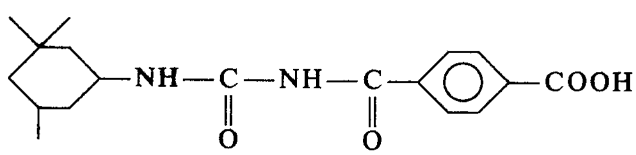 |
| 55 | 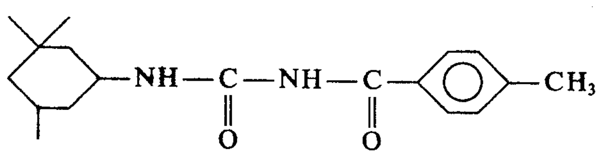 |

Fortsetzung

| Beispiel Nr. | Chemische Struktur |
|---|---|
| 56 | 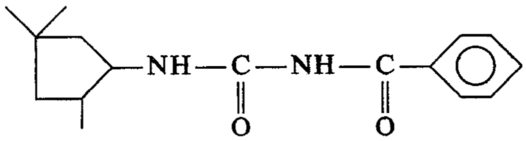 |
| 57 | 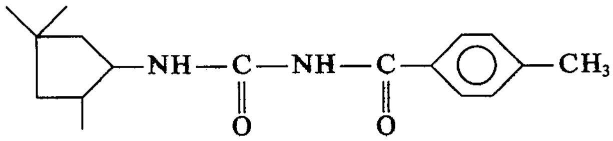 |
| 58 | 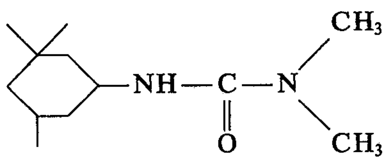 |
| 59 | 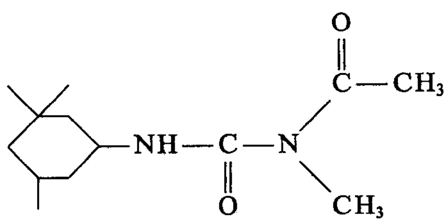 |
| 60 | 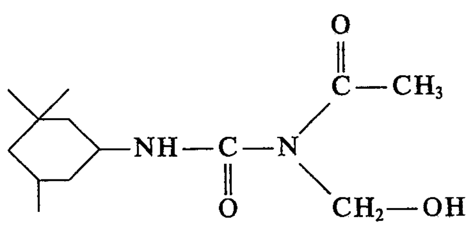 |
| 61 | 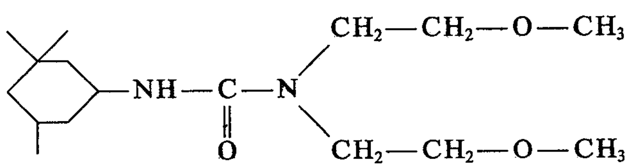 |
| 62 | 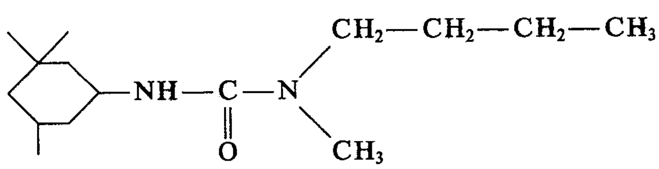 |
| 63 | 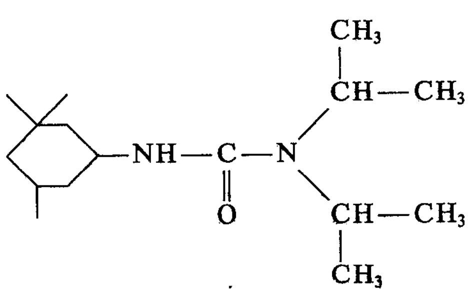 |

Fortsetzung

| Beispiel Nr. | Chemische Struktur |
|---|---|
| 64 | 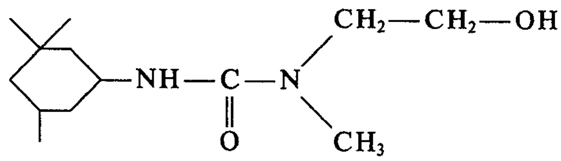 |
| 65 | 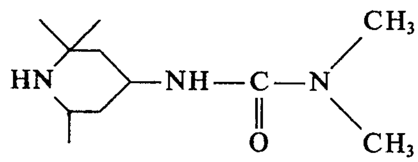 |
| 66 | 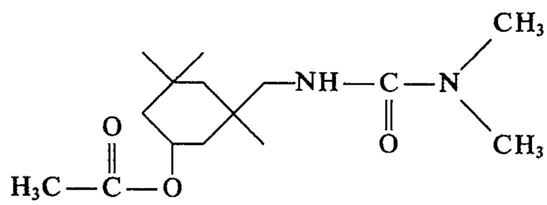 |
| 67 | 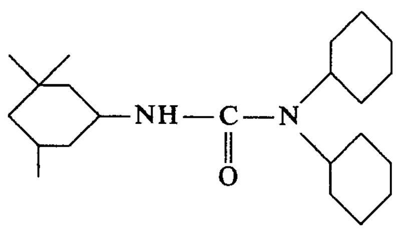 |
| 68 | 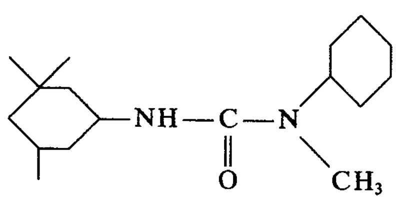 |
| 69 | 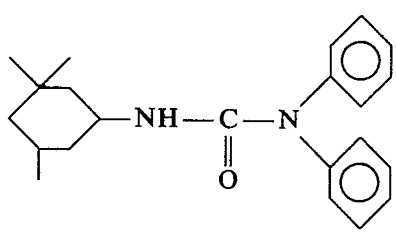 |
| 70 | 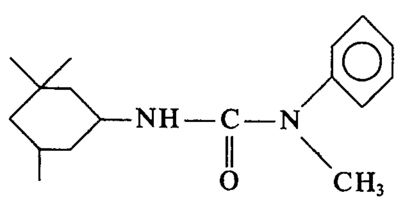 |
| 71 | 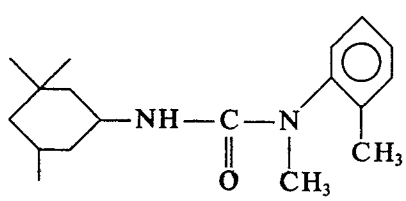 |

Fortsetzung

| Beispiel Nr. | Chemische Struktur |
|---|---|
| 72 | 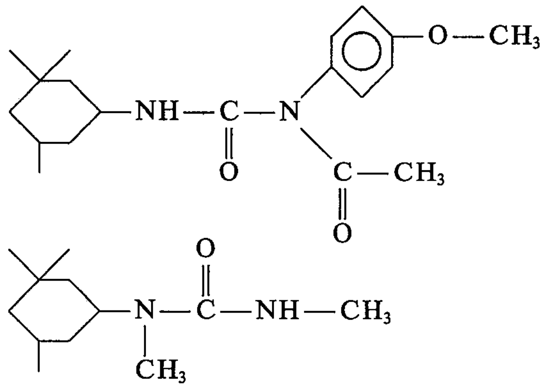 |
| 73 | |
| 74 | 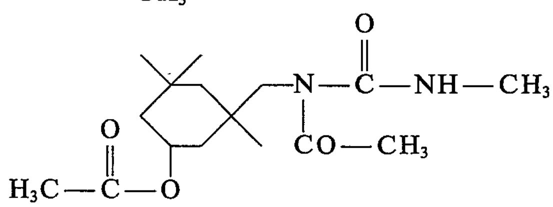 |
| 75 | 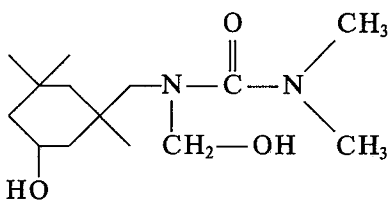 |
| 76 | 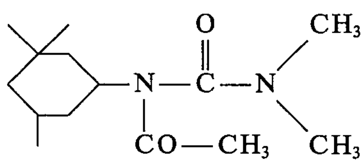 |
| 77 | 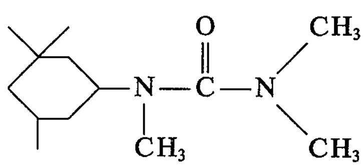 |
| 78 | 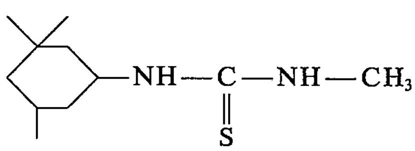 |
| 79 | 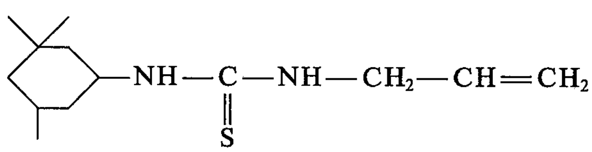 |
| 80 | 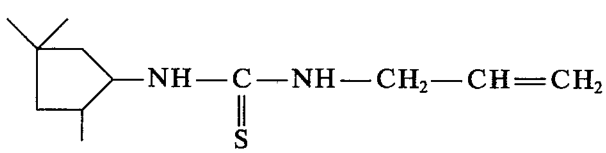 |

Fortsetzung

| Beispiel Nr. | Chemische Struktur |
|---|---|
| 81 | 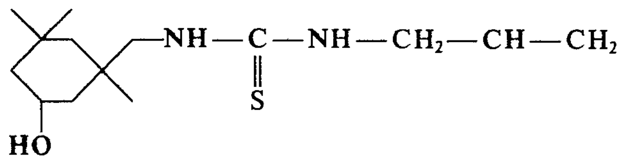 |
| 82 | 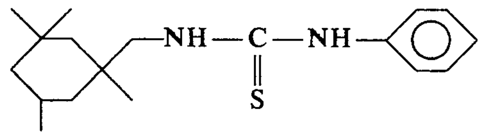 |
| 83 | 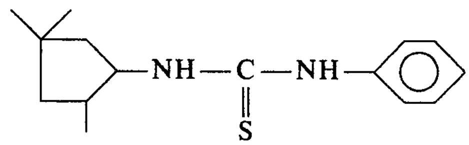 |
| 84 | 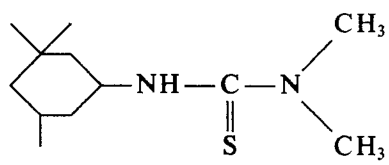 |
| 85 | 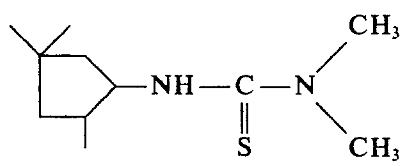 |
| 86 | 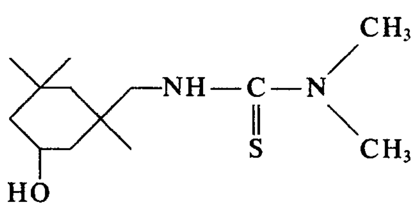 |

Nachstehend wird die Erfindung durch Beispiele repräsentativ für die Herstellung der Verbindungen gemäß der angegebenen allgemeinen Formel I näher erläutert.

Herstellungsbeispiel 1

N-(3,3,5-Trimethyl-cyclohexyl)-harnstoff

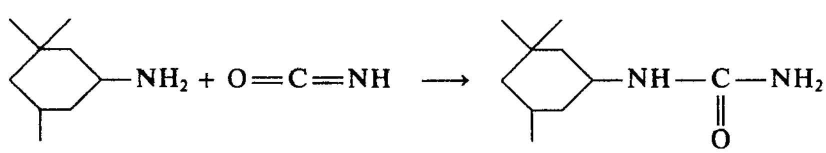

0,5 Mol 3,3,5-Trimethyl-cyclohexylamin wurden in 240 ml Eisessig und 100 ml Wasser bei 40°C gelöst. Hierzu wurde eine Lösung von 1 Mol Natriumcyanat in 100 ml Wasser bei 40°C gesetzt. Etwa 1/10 dieser Lösung wurde langsam unter Rühren zugetropft, bis ein weißer kristalliner Niederschlag auftrat, der Rest wurde dann sehr schnell bei intensiver Rührung zugefügt. Bei der Reaktion stieg die Temperatur auf ca. 55°C. Der ausgeschiedene Brei wurde noch eine Viertelstunde gerührt und 2–3

Stunden bei Raumtemperatur stehengelassen, mit Wasser verdünnt, auf 0° abgekühlt und filtriert, mit Wasser gewaschen und anschließend getrocknet.

Ausbeute: 84,5 g, weißes kristallines Pulver, 92% d. Th.

Im wäßrigen Aceton umkristallisiert erhält man ein sehr reines Produkt

Analyse:

| | | | |
|---|---|---|---|
| gef. | C 65,3 | H 10,7 | N 15,5% |
| ber. | C 65,23 | H 10,86 | N 15,2% |
| Fp. | 182 – 184° C | | |

Herstellungsbeispiel 2

N-(3,3,5-Trimethylcyclohexyl)-N′,N′-dimethylharnstoff

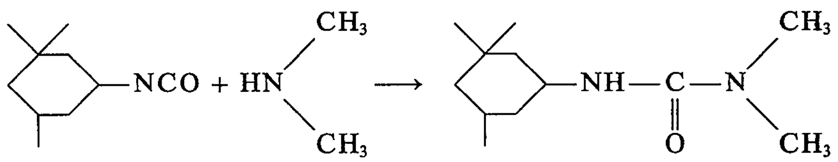

0,25 Mol ≙ 41,7 g 3,3,5-Trimethylcyclohexylisocyanat in 50 ml Benzol gelöst, wurden zu einer Lösung von 0,25 Mol ≙ 10,2 g Dimethylamin in 100 ml Benzol unter lebhaftem Rühren gegeben. Die Mischung wurde 1 h am Rückfluß erhitzt. Nach dem Erkalten wurde die Reaktionslösung mit 2 n wäßriger Salzsäure, 2 n wäßriger Natronlauge und Wasser in der angegebenen Reihenfolge gewaschen und die organische Phase über Natriumsulfat getrocknet. Durch Abdampfen des Lösungsmittels wurde die Lösung zur Gewinnung von Kristallen konzentriert, die aus n-Hexan zur Gewinnung des in der Überschrift angegebenen Harnstoffs als Kristalle umkristallisiert wurden.

Ausbeute: 51,2 g, 98,7% der Theorie.

Analyse:

| | | | |
|---|---|---|---|
| gef. | C 67,4 | H 11,5 | N 13,4% |
| ber. | C 67,94 | H 11,31 | N 13,21% |
| Fp. | 85 – 87° C | | |

Herstellungsbeispiel 3

N-3,3,5-Trimethylcyclohexyl)-N′-methoxyharnstoff

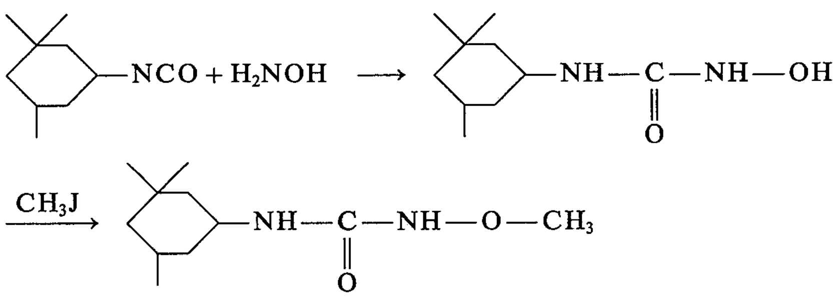


Eine Lösung von 0,25 Mol ≙ 8,3 g Hydroxylamin in 50 ml Benzol wurde zu einer Lösung von 0,25 Mol ≙ 41,7 g 3,3,5-Trimethylcyclohexylisocyanat in 50 ml Benzol gegeben. Die Mischung wurde gut gerührt und über Nacht stehengelassen. Die ausgefällten Kristalle wurden abfiltriert und mit n-Hexan zur Gewinnung von N-(3,3,5-Trimethylcyclohexyl)-N′-hydroxyharnstoff gewaschen.

Ausbeute: 49,6 g, 99,2% der Theorie.

Zu 0,2 Mol ≙ 40 g dieses Harnstoffs wurde eine Lösung von 54 g Natriummethoxid in 100 ml Methanol gegeben. Die Mischung wurde durch Rühren homogenisiert und mit 0,2 Mol ≙ 28,4 g Methyljodid versetzt, danach unter Rückfluß 2 h lang erhitzt. Das Lösungsmittel wurde abgedampft und der Rückstand mit Benzol nach Wasserzugabe extrahiert. Die benzolische Lösung wurde über

Natriumsulfat getrocknet und zur Gewinnung von Kristallen eingeengt, die aus einer Mischung von n-Hexan und Benzol zur Gewinnung der in der Uberschrift angegebenen Verbindung umkristallisiert wurden.

Ausbeute: 41,8 g, 97,6% der Theorie.

Analyse:

| | | | |
|---|---|---|---|
| gef. | C 62 | H 9,9 | N 13,1% |
| ber. | C 61,69 | H 10,27 | N 13,08% |

Herstellungsbeispiel 4

N-(3,3,5-Trimethylcyclohexyl)-N-methyl-N',N'-dimethylharnstoff

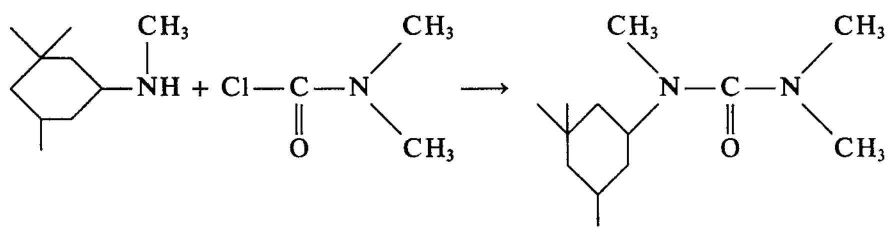

Zu einer Lösung von 0,5 Mol ≙ 77,6 g N-Methyl-3,3,5-Trimethylcyclohexylamin in 100 ml Benzol wurde eine Lösung von 0,25 Mol ≙ 26,9 g N,N-Dimethylcarbamoylchlorid, gelöst in 100 ml Benzol, gegeben. Die Temperatur der Mischung stieg bei der Reaktion von Raumtemperatur auf 80° C an und wurde 1 h lang bei 80° C gehalten. Das Aminhydrochlorid wurde dann mit Wasser ausgeschüttelt, die benzolische Lösung abgetrennt, über Natriumsulfat getrocknet und eingeengt. Aus der konzentrierten Lösung fiel ein weißer kristalliner Niederschlag aus.

Ausbeute des in der Überschrift angegebenen Harnstoffs:

53,6 g, 94,8% der Theorie.

Analyse:

| | | | |
|---|---|---|---|
| gef. | C 69,4 | H 11,2 | N 12,2% |
| ber. | C 69,04 | H 11,5 | N 12,39% |

Die wäßrige Phase, in der das Aminhydrochlorid gelöst ist, wurde zur Rückgewinnung des Amins mit der äquivalenten Menge wäßriger Natronlauge versetzt, und das freigesetzte Amin mit Benzol mehrmals extrahiert. Nach fraktionierter Destillation erhält man das zur Säurebindung gediente Amin fast quantitativ zurück.

Herstellungsbeispiel 5

N-(2,4,4-Trimethylcyclopentyl)-N'-phenyl-thioharnstoff

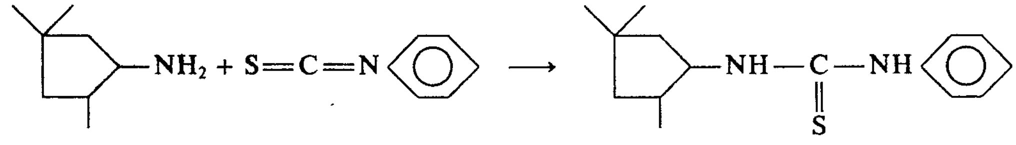

Eine Lösung von 0,2 Mol ≙ 25,4 g 2,4,4-Trimethylcyclopentylamin in 50 ml Xylol wurde zu einer Lösung von 0,2 Mol ≙ 27 g Phenylsenföl in 50 ml Xylol gegeben und mit einer Spatelspitze Dibutylzinndilaurat als Katalysator versetzt. Die Mischung ließ man 6 h am Rückfluß kochen, danach wurde die Reaktionslösung mit 2 n wäßriger Salzsäure, 2 n wäßrigem Natriumhydroxid und Wasser in der angegebenen Reihenfolge gewaschen und die organische Phase über Natriumsulfat getrocknet. Anschließendes Abdampfen des Lösungsmittels ergab aus der konzentrierten Lösung einen kristallinen Niederschlag, der abfiltriert und aus einer Mischung Benzol/n-Hexan umkristalliert wurde.

Ausbeute: 49,4 g, 94,2% der Theorie.

Analyse:

| | | | | |
|---|---|---|---|---|
| gef. | C 69,01 | H 8,7 | N 10,3 | S 12,1% |
| ber. | C 68,69 | H 8,39 | N 10,68 | S 12,24% |

Herstellungsbeispiel 6

N-(1,3,3-Trimethyl-5-hydroxy-cyclohexyl)-methyl-N',N'-dimethylthioharnstoff

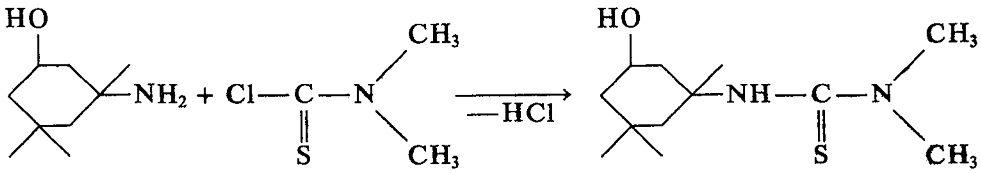


Eine Lösung von 0,5 Mol ≙ 85,5 g 1-Aminomethyl-3-hydroxy-1,5,5-trimethyl-cyclohexan in 150 ml Toluol wurde tropfenweise mit einer Lösung von 0,25 Mol ≙ 30,9 g N,N-Dimethylthiocarbamidsäurechlorid in 100 ml Toluol versetzt, hierbei stieg die Reaktionstemperatur auf 80°C an. Man ließ 1 1/2 h am Rückfluß kochen. Das zur Säurebindung eingesetzte überschüssige Amin wurde als Hydrochlorid mit Wasser extrahiert und separat wieder zurückgewonnen, die organische Phase getrocknet und bis zur Kristallisation des in der Überschrift angegebenen Thioharnstoffs konzentriert.

Ausbeute: 62,1 g, 96,2% der Theorie.

Analyse:

| | | | | |
|---|---|---|---|---|
| gef. | C 61 | H 10,3 | N 10,7 | S 12,8% |
| ber. | C 60,46 | H 10,07 | N 10,85 | S 12,43% |

Es wurde weiterhin festgestellt, daß bestimmte Harnstoffverbindungen einer Struktur, in der wenigstens ein alicyclischer oder heterocyclischer Rest mit mindestens drei Substituenten — davon bevorzugt zwei in geminaler Stellung — vorhanden ist, insbesondere als selektive Unkrautbekämpfungsmittel zu verwenden sind. Die Struktur der Harnstoffverbindungen gemäß der Erfindung ist neu und klar von der bekannter Harnstoff-Unkrautbekämpfungsmittel verschieden.

Die Verbindungen der allgemeinen Formel I besitzen eine hervorragend hohe Selektivität u. a. in Winterweizen, Wintergetreide, Roggen, Sommergerste und Sommerweizen. So sind diese Verbindungen sehr gut wirksam u. a. gegen Ackerfuchsschwanz, Windhalm, Vogelmiere und Kamille.

Die Kulturverträglichkeit in Sommergetreide (Hafer, Sommergerste, Sommerweizen etc.) ist besser als die aller Vergleichsmittel. Gerade bei Sommergerste und Hafer fehlen wirksame Bodenherbizide mit Ackerfuchsschwanz- und vor allem Windhalmwirkung. Diese Aufgabe wird durch die erfindungsgemäßen Verbindungen ausgezeichnet gelöst. Bei Sommerweizen und Sommergerste ist eine Anwendung sowohl im Vorauflauf- (VA) als auch im Nachauflaufverfahren (NA) geeignet. Im Nachauflaufverfahren ist zudem der Vorteil gegeben, daß eine Anwendung in Kombination mit anderen Herbiziden (z. B. Phenoxyfettsäuren, Triazinen) durchgeführt werden kann.

Im Hinblick auf die Beziehung Struktur/Aktivität der Verbindungen der Erfindung ist das vollständig gesättigte und hochsubstituierte Ringsystem für die selektive herbizide Aktivität wesentlich. Der Rest A der allgemeinen Formel (I) spielt eine große Rolle hinsichtlich der Aktivität, besonders dann, wenn zwei Methylreste geminalständig sind. Ersetzt man einen geminalen Rest — bei niedriger Substitution z. B. drei — durch ein Wasserstoffatom, so wird die Aktivität deutlich herabgesetzt.

Darüber hinaus zeichnen sich die erfindungsgemäßen Verbindungen durch wachstumsregulatorische Eigenschaften aus, die sich in einer verlängerten Reifezeit des Getreides äußern. Die hiermit verlängerte Assimilateinlagerung in das reifende Korn korreliert u. a. mit höheren Eiweißgehalten im Getreidekorn. Ein wesentlicher Faktor für die Wirksamkeit der Verbindungen entsprechend der allgemeinen Formel I ist der Raumbedarf (molecular bulkiness) des Moleküls.

So tritt ganz überraschend dieser wachstumsregulatorische Effekt besonders stark bei den Verbindungen auf, die am Cyclohexylgerüst trans konformiert sind und bei denen $\gamma=0$ entsprechend der allgemeinen Formel (I) ist.

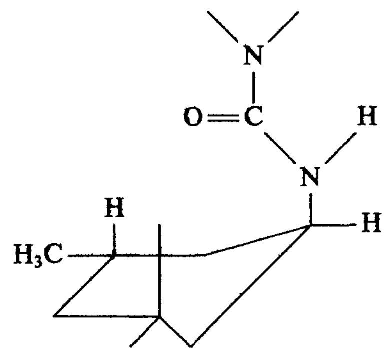

trans (ep, ax)

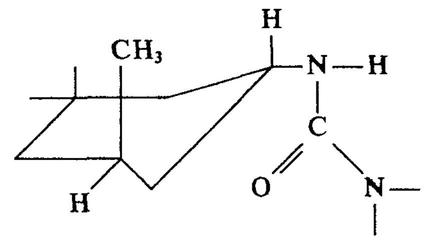

trans (as, eq)

Die erfindungsgemäßen Verbindungen der allgemeinen Formel I, die im Vor- und Nachauflaufverfahren gute herbizide Wirksamkeit gegen eine Reihe wichtiger Schadgräser zeigen, werden andererseits auch bei hohen Dosierungen durch dikotyle Kulturpflanzen und verschiedene Getreidearten gut toleriert. Sie eignen sich in hervorragender Weise als Antidots giftiger herbizider Mittel und als Komponente im Gemisch mit bekannten und bedeutenden Herbiziden aus der Klasse der Triazine, Harnstoffe und Phenoxyfettsäuren ausgezeichnet zur Herstellung von Breitbandherbiziden mit synergistischer Wirkung.

Ein großer Vorteil der erfindungsgemäßen Verbindungen liegt neben ihren überraschend außergewöhnlichen Wirkungseffekten und Anwendungsgebieten ferner darin, daß sie überhaupt kein Halogen und schwer abbaubare Gruppen enthalten, die zu Rückstandsproblemen im Boden oder in der Pflanze führen.

Infolge der Verträglichkeit durch die Kulturpflanzen, der raschen Wirkungsweise, des damit verbundenen schnellen Abbaus der Wirkstoffe gemäß der angegebenen allgemeinen Formel (I) und dadurch, daß Rückstandsprobleme im Boden oder in der Kulturpflanze nicht auftreten, sind ökologische Bedenken — nach dem gegenwärtigen Stande der Technik — nicht gegeben.

Die Wirkstoffe gemäß der allgemeinen Formel (I) können im allgemeinen zu 2—95 Gew.-% neben anderen Herbizidkomponenten enthalten sein und in Form verschiedener Zubereitungen vorgesehen werden.

So können die Wirkstoffe in die üblichen Formulierungen übergeführt werden, wie z. B. Lösungen, Emulsionen, Spritzpulver, Suspensionen, Pulver, Stäubemittel, Schäume, Pasten, lösliche Pulver, Granulate, Aerosole, Suspensions-, Emulsionskonzentrate, Saatgutpuder, Wirkstoff-imprägnierte Natur- und synthetische Stoffe sowie Feinstverkapselungen in polymeren Stoffen und in Hüllmassen für Saatgut.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten wie Xylol, Toluol oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glykol sowie deren Äther und Ester, Ketone wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Buten, Propan, Stickstoff und Kohlendioxid; als feste Trägerstoffe: natürliche Gesteinsmehle wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel: nicht ionogene und anionische Emulgatoren, wie Polyoxyäthylen-Fettsäure-Ester, Polyoxyäthylen-Fettalkohol-Äther, z. B. Alkylaryl-polyglykol-äther, Alkylsulfonate, Alkylsulfate, Arylsulfonate sowie Eiweißhydrolysate; als Dispergiermittel: z. B. Lignin, Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin, Azo-Metall-phthalocyaninfarbstoffe und Spurennährstoffe wie Salze von Eisen, Mangan, Bor, Kupfer, Cobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-%, liegen. Die Anwendung geschieht in einer den Anwendungsformen angepaßten Weise.

Die nachfolgenden Beispiele erläutern die Erfindung: die Aufbereitung der Wirkstoffe für eine praktische Anwendung und die erzielten Wirkungen bei der biologischen Prüfung.

Ein in Wasser leicht dispergierbares benetzbares Pulver wird erhalten, indem man

70 Teile Wirkstoff
25 Teile hochdisperse Kieselsäure
3 Teile Emulgator, bestehend aus anionischen und nicht ionischen Tensiden
2 Teile Polyphosphat als Netz- und Dispergiermittel und als Komplexbildner

mischt und in einer Mühle mahlt.

Das Mittel wird dann vor der Anwendung auf die entsprechende Gebrauchskonzentration in Wasser dispergiert und als Spritzpulver in Einsatz gebracht.

## Herbizide Wirkungen bei Applikation der Wirkstoffe

### Anwendungsbeispiel 1

Im Gewächshaus wurden die in der nachfolgenden Tabelle 1 aufgelisteten Verbindungen als Prototypen der erfindungsgemäßen Verbindungen in einer Aufwandmenge von 4 kg Wirkstoff/ha bezogen auf reinen Wirkstoff, suspendiert in 1200 l Wasser/ha, auf folgende Pflanzen im Vorauflauf (VA) appliziert auf die Bodenoberfläche vor dem Keimen der Samen, und im Nachauflauf (NA) auf die Blattoberfläche gespritzt, bei Dikotyledonen im 1. Laubblattstadium und bei Monokotyledonen im 3. Blattstadium. Drei Wochen nach der Behandlung wurde die Wirkung der Präparate bonitiert. In den Tabellen bedeutet die Bonitierungsnote 1 volle Wirkung; Vernichtung der Pflanzen; die Bonitierungsnote 5 keine Wirkung, Pflanzen wie unbehandelt. Tabelle 2 zeigt Vergleichsmittel.

Tabelle 1

| Erfindungsgemäße Mittel | Tomate | | Ackersenf | | Hafer | | Flughafer | | Ackerfuchs-schwanz | |
|---|---|---|---|---|---|---|---|---|---|---|
| | VA | NA | VA | NA | VA | NA | VA | NA | VA | NA |
| 1-(3,3,5-Trimethylcyclohexyl)-3,3-dimethylharnstoff (cis-Form) | 1 | 1 | 1 | 1 | 2,5 | 4 | 2 | 3,5 | 1 | 1 |
| 1-(3,3,5-Trimethylcyclohexyl)-3,3-dimethylharnstoff (trans-Form) | 1 | 1 | 1 | 1 | 2 | 2 | 1 | 1,5 | 1 | 1 |
| 1-(3,3,5-Trimethylcyclohexyl)-3,3-dimethylthioharnstoff (cis-Form) | 1 | 1 | 1 | 1 | 2 | 3 | 2 | 2 | 1 | 1 |

Die Ergebnisse mit den erfindungsgemäßen Verbindungen zeigen drei Wochen nach der Behandlung eine gute Wirkung im Vorauflauf- wie im Nachauflaufverfahren bei den getesteten Dikotyledonen, die cis-Form der v. g. Verbindung eine gute Kulturverträglichkeit im Nachauflauf bei Hafer.

Tabelle 2

| Vergleichsmittel | Tomate | | Ackersenf | | Hafer | | Flughafer | | Ackerfuchs-schwanz | |
|---|---|---|---|---|---|---|---|---|---|---|
| | VA | NA | VA | NA | VA | NA | VA | NA | VA | NA |
| 1-Cyclohexyl-3,3-dimethyl-harnstoff | 1 | 1 | 1 | 1 | 1 | 1 | 1 | 1 | 1 | 1 |
| 1-(3-Chlor-4-methyl-phenyl)-3,3-dimethylharnstoff (Dicuran®) | 1 | 1 | 1 | 1 | 1 | 2 | 1 | 1 | 1 | 1 |

Botanische Namen der verwendeten Pflanzen:

| | |
|---|---|
| Tomate: | Solanum lycopersicum |
| Ackersenf: | Sinapsis alba |
| Hafer: | Avena sativa |
| Flughafer: | Avena futua |
| Ackerfuchsschwanz: | Alopecurus myosoroides |

Anwendungsbeispiel 2

Im Gewächshaus wurden die in den nachfolgenden Tabellen 3 und 4 aufgeführten erfindungsgemäßen Verbindungen in Aufwandmengen von 4, 3, 2 und 1 kg Wirkstoff/ha bezogen auf reinen Wirkstoff, suspendiert in 1200 l Wasser/ha, im Vorauflauf (VA) und im Nachauflauf (NA) an den genannten verschiedenen Pflanzen geprüft.

Tabellen 5 und 6 zeigen Vergleichsmittel.

Legende: sonst wie in Tabelle 1.

Tabelle 3

| Testpflanzen | | Herbizide Wirkung von 1-(3,3,5-Trimethyl-cyclohexyl)-3,3-dimethyl-harnstoff, cis-Form Aufwandmengen in kg Wirkstoff/ha | | | |
|---|---|---|---|---|---|
| | | 1 | 2 | 3 | 4 |
| Ackersenf | VA | 1 | 1 | 1 | 1 |
| | NA | 1 | 1 | 1 | 1 |
| Tomate | VA | 1 | 1 | 1 | 1 |
| | NA | 1 | 1 | 1 | 1 |
| Hühnerhirse | VA | 3 | 2 | 1 | 1 |
| | NA | 4 | 2 | 1 | 1 |
| Hafer | VA | 5 | 3 | 2,5 | 2,5 |
| | NA | 5 | 3 | 1,5 | 1 |
| Flughafer | VA | 2 | 1 | 1 | 1 |
| | NA | 3,5 | 2 | 1 | 1 |
| Ackerfuchsschwanz | VA | 2 | 1 | 1 | 1 |
| | NA | 1 | 1 | 1 | 1 |
| Vogelmiere | VA | 2 | 1 | 1 | 1 |
| | NA | 2 | 1,5 | 1 | 1 |
| Kornblume | VA | 4 | 2,5 | 1 | 1 |
| | NA | 4 | 4 | 2 | 1 |
| Kamille | VA | 1 | 1 | 1 | 1 |
| | NA | 1,5 | 1 | 1 | 1 |
| Weißer Gänsefuß | VA | 1 | 1 | 1 | 1 |
| | NA | 3 | 1 | 1 | 1 |
| Windhalm | VA | 1 | 1 | 1 | 1 |
| | NA | 1 | 1 | 1 | 1 |
| Sommergerste | VA | 4 | 4 | 3 | 1,5 |
| | NA | 3 | 3 | 3 | 2,5 |
| Sommerweizen | VA | 4,5 | 4 | 3 | 1,5 |
| | NA | 3 | 2 | 1,5 | 1 |
| Sojabohne | VA | 4,5 | 4,5 | 3 | 2,5 |
| | NA | – | – | – | – |
| Baumwolle | VA | 3 | 3 | 3 | 3 |
| | NA | 5 | 5 | 4,5 | 4 |

Tabelle 4

| Testpflanzen | | Herbizide Wirkung von 1-3,3,5-Trimethylcyclohexyl)-3,3-dimethylharnstoff, trans-Form Aufwandmengen kg Wirkstoff/ha | | | |
|---|---|---|---|---|---|
| | | 1 | 2 | 3 | 4 |
| Ackersenf | VA | 1 | 1 | 1 | 1 |
| | NA | 1 | 1 | 1 | 1 |
| Tomate | VA | 1 | 1 | 1 | 1 |
| | NA | 1 | 1 | 1 | 1 |
| Hühnerhirse | VA | 2 | 1 | 1 | 1 |
| | NA | 3 | 2 | 1 | 1 |
| Hafer | VA | 2 | 2 | 2,5 | 1 |
| | NA | 2 | 2 | 2 | 1 |
| Flughafer | VA | 1 | 1 | 1 | 1 |
| | NA | 1,5 | 1 | 1 | 1 |
| Ackerfuchsschwanz | VA | 1 | 1 | 1 | 1 |
| | NA | 1 | 1 | 1 | 1 |
| Vogelmiere | VA | 1 | 1 | 1 | 1 |
| | NA | 1 | 1 | 1 | 1 |
| Kornblume | VA | 3 | 1 | 1 | 1 |
| | NA | 3 | 3 | 2 | 1 |
| Kamille | VA | 1 | 1 | 1 | 1 |
| | NA | 1 | 1 | 1 | 1 |
| Weißer Gänsefuß | VA | 1 | 1 | 1 | 1 |
| | NA | 1 | 1 | 1 | 1 |
| Windhalm | VA | 1 | 1 | 1 | 1 |
| | NA | 1 | 1 | 1 | 1 |
| Sommergerste | VA | 4 | 4 | 3 | 1 |
| | NA | 3 | 3 | 3 | 1 |
| Sommerweizen | VA | 4 | 4 | 2 | 1,5 |
| | NA | 3 | 3 | 1 | 1 |
| Sojabohne | VA | 4,5 | 4 | 3,5 | 3,5 |
| | NA | – | – | – | – |
| Baumwolle | VA | 2 | 2 | 1 | 1 |
| | NA | 4 | 4 | 4 | 2 |

Die erfindungsgemäßen Verbindungen zeigen in den unteren Dosierungen eine ausgezeichnete Verträglichkeit bei Sommergetreide (Sommergerste, Sommerweizen, Hafer).

Tabelle 5

– Vergleichsmittel –

| Testpflanzen | | Herbizide Wirkung von 1-Cyclohexyl-3,3-dimethylharnstoff Aufwandmengen in kg Wirkstoff/ha | | | |
|---|---|---|---|---|---|
| | | 1 | 2 | 3 | 4 |
| Ackersenf | VA | 1 | 1 | 1 | 1 |
| | NA | 1 | 1 | 1 | 1 |
| Tomate | VA | 1 | 1 | 1 | 1 |
| | NA | 1 | 1 | 1 | 1 |
| Hühnerhirse | VA | 2 | 2 | 1 | 1 |
| | NA | 4 | 4 | 1 | 1 |
| Hafer | VA | 2 | 1 | 1 | 1 |
| | NA | 3 | 3 | 1,5 | 1 |
| Flughafer | VA | 2 | 1 | 1 | 1 |
| | NA | 2 | 1 | 1 | 1 |
| Ackerfuchsschwanz | VA | 1 | 1 | 1 | 1 |
| | NA | 1 | 1 | 1 | 1 |
| Vogelmiere | VA | 2 | 1 | 1 | 1 |
| | NA | 2 | 1,5 | 1 | 1 |
| Kornblume | VA | 4 | 2 | 1 | 1 |
| | NA | 3 | 3 | 2 | 1 |
| Kamille | VA | 1 | 1 | 1 | 1 |
| | NA | 1 | 1 | 1 | 1 |
| Weißer Gänsefuß | VA | 1 | 1 | 1 | 1 |
| | NA | 1 | 1 | 1 | 1 |
| Windhalm | VA | 1 | 1 | 1 | 1 |
| | NA | 1 | 1 | 1 | 1 |
| Sommergerste | VA | 2,5 | 2,5 | 2 | 1 |
| | NA | 4 | 4 | 3 | 1 |
| Sommerweizen | VA | 2 | 2,5 | 1,5 | 1,5 |
| | NA | 4 | 3 | 1 | 1 |
| Sojabohne | VA | 5 | 4,5 | 3,5 | 3,5 |
| | NA | – | – | – | – |
| Baumwolle | VA | 2,5 | 1 | 1 | 1 |
| | NA | 4 | 1 | 1 | 1 |

Tabelle 6

— Vergleichsmittel —

| Testpflanzen | | Herbizide Wirkung von 1-(3-Chlor-4-methyl-phenyl)-3,3-dimethyl-harnstoff, Dicuran* Aufwandmengen in kg Wirkstoff/ha | | | |
|---|---|---|---|---|---|
| | | 1 | 2 | 3 | 4 |
| Ackersenf | VA | 1 | 1 | 1 | 1 |
| | NA | 1 | 1 | 1 | 1 |
| Tomate | VA | 1 | 1 | 1 | 1 |
| | NA | 1 | 1 | 1 | 1 |
| Hühnerhirse | VA | 1,5 | 1 | 1 | 1 |
| | NA | 2 | 1 | 1 | 1 |
| Hafer | VA | 1 | 1 | 1 | 1 |
| | NA | 1 | 1 | 1 | 1 |
| Flughafer | VA | 2,5 | 1 | 1 | 1 |
| | NA | 1,5 | 1 | 1 | 1 |
| Ackerfuchsschwanz | VA | 1 | 1 | 1 | 1 |
| | NA | 1 | 1 | 1 | 1 |
| Vogelmiere | VA | 1 | 1 | 1 | 1 |
| | NA | 1 | 1 | 1 | 1 |
| Kornblume | VA | 2,5 | 1 | 1 | 1 |
| | NA | 3 | 1 | 1 | 1 |
| Kamille | VA | 1 | 1 | 1 | 1 |
| | NA | 1 | 1 | 1 | 1 |
| Weißer Gänsefuß | VA | 1 | 1 | 1 | 1 |
| | NA | 1 | 1 | 1 | 1 |
| Windhalm | VA | 1 | 1 | 1 | 1 |
| | NA | 1 | 1 | 1 | 1 |
| Sommergerste | VA | 1,5 | 1 | 1 | 1 |
| | NA | 1,5 | 1 | 1 | 1 |
| Sommerweizen | VA | 2 | 1,5 | 1 | 1 |
| | NA | 2 | 1 | 1 | 1 |
| Sojabohne | VA | 2 | 1 | 1 | 1 |
| | — | — | — | — | — |
| Baumwolle | VA | 3 | 3 | 2 | 1 |
| | NA | 3 | 2 | 1 | 1 |

Botanische Namen der verwendeten Pflanzen:

| | |
|---|---|
| Ackersenf: | Sinapsis alba |
| Tomate: | Solanum lycopersicum |
| Hüherhirse: | Echinochloa crus-galli |
| Hafer: | Avena sativa |
| Flughafer: | Avena fatua |
| Ackerfuchsschwanz: | Alopecurus myosoroides |
| Vogelmiere: | Stellaria media |
| Kornblume: | Centaurea cyanus |
| Kamille: | Anthemis spec. |
| Weißer Gänsefuß: | Chenoposium album |
| Windhalm: | Apera spica-venti |
| Gerste: | Hordeum vulgare |
| Weizen: | Triticum aestivum |
| Sojabohne: | Soja hispida |
| Baumwolle: | Gossypium hirsutum |

**Patentansprüche**

1. Harnstoffe mit cyclischen Substituenten der folgenden allgemeinen Formel I

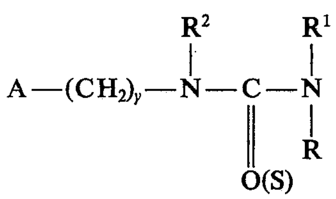

(I)

in der A =

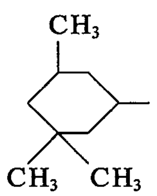

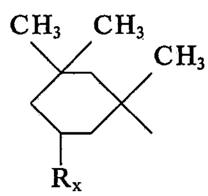

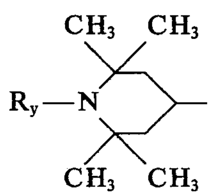

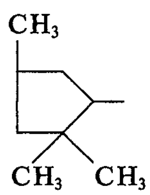

und

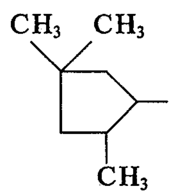

einschließlich der Stellungsisomeren, insbesondere der trans-Form mit

$R_x$ = OH, Carboxyl;
$R_y$ = H, Acyl;
R = Wasserstoff, Hydroxyl, Alkyoxyl oder Acyl, einen $C_1-C_{12}$-Alkylrest gegebenenfalls mit Hydroxylgruppen, der geradkettig oder verzweigt und durch Sauerstoff und/oder Stickstoff-Atome unterbrochen sein kann, einen $C_3-C_{12}$-Cycloalkylrest gegebenenfalls mit Alkylsubstituenten, die durch Sauerstoff- und/oder Stickstoff-Atome unterbrochen sein können, und/oder mit Hydroxyl- und/oder mit einem aliphatischen oder aromatischen Acyl- und/oder mit Carboxyl- oder Carbalkoxygruppen,
einen $C_2-C_5$-Alkenyl- oder Alkinylrest,
einen Phenylrest, der durch Alkyl- und/oder Acyl- und/oder Hydroxyl- und/oder Dialkylaminogruppen substituiert sein kann;
$R^1$ = Wasserstoff oder einen geradkettigen oder verzweigten $C_1-C_4$-Alkylrest oder Alkoxyrest;
$R^2$ = Wasserstoff oder einen geradkettigen oder verzweigten $C_1-C_4$-Alkylrest gegebenenfalls mit Hydroxylgruppen, oder $C_1-C_4$-Acylrest und
$\gamma$ = eine Zahl von 0 bis 1

bedeuten.

2. Harnstoffe mit cyclischen Substituenten nach Anspruch 1, dadurch gekennzeichnet, daß mindestens zwei Substituenten am Ring trans-ständig sind.

3. Verfahren zur Herstellung der Harnstoffe mit cyclischen Substituenten nach den Ansprüchen 1 und 2, dadurch gekennzeichnet, daß man die entsprechenden Amine mit den entsprechenden Isocyanaten bzw. Thioisocyanaten oder Carbamoylchloriden umsetzt.

4. Verwendung einer oder mehrerer Harnstoffe mit cyclischen Substituenten nach den Ansprüchen 1 und 2 als Herbizide.

5. Verwendung einer oder mehrerer Harnstoffe mit cyclischen Substituenten nach Anspruch 1 in Kombination mit anderen Herbiziden.

6. Verwendung der Herbizide nach den Ansprüchen 1, 2, 4 und 5 im Gemisch mit inerten Trägern.

**Claims**

1. Ureas, with cyclic substituents, of the following general formla I

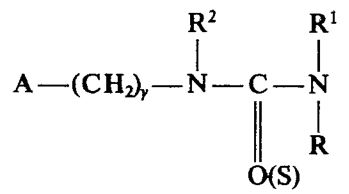

(I)

in which A =

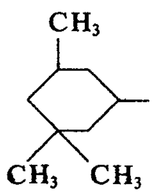

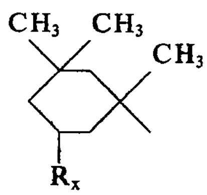

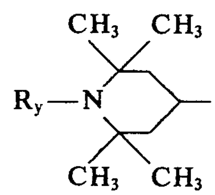

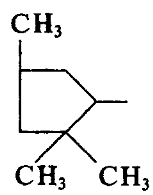

and

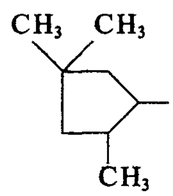

including the positional isomers, in particular the transform with

$R_x$ denoting OH or carboxyl;

$R_y$ denoting H or acyl;

R denoting hydrogen, hydroxyl, alkyloxy or acyl, a $C_1-C_{12}$-alkyl radical, optionally with hydroxyl groups, which can be straight-chain or branched and interrupted by oxygen and/or nitrogen atoms, a $C_3-C_{12}$-cycloalkyl radical, optionally with alkyl substituents, which can be interrupted by oxygen and/or nitrogen atoms, and/or with hydroxyl groups and/or with an aliphatic or aromatic acyl group and/or with carboxyl or carboalkoxy groups, a $C_2-C_5$-alkenyl or alkenyl radical, or a phenyl radical which can be substituted by alkyl and/or acyl and/or hydroxyl and/or dialkylamino groups;

$R^1$ denotes hydrogen or a straight-chain or branched $C_1-C_4$-alkyl radical or alkoxy radical;

$R^2$ denotes hydrogen or a straight-chain or branched $C_1-C_4$-alkyl radical, optionally with hydroxyl groups, or $C_1-C_4$-acyl radical and

$\gamma$ denotes a number form 0 to 1.

2. Ureas with cyclic substituents according to Claim 1, characterised in that at least two substituents on the ring are trans.

3. Process for the preparation of the ureas with cyclic substituents according to Claims 1 and 2, characterised in that the appropriate amines are reacted with the appropriate isocyanates or thioisocyanates or carbamoyl chlorides.

4. Use of one or more ureas with cyclic substituents according to Claims 1 and 2 as herbicides.

5. Use of one of more ureas with cyclic substituents according to Claim 1 in combination with other herbicides.

6. Use of the herbicides according to Claims 1, 2, 4 and 5 in a mixture with inert vehicles.

**Revendications**

1. Urées avec substituants cycliques de formule générale I

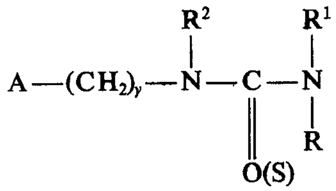 (I)

dans laquelle A =

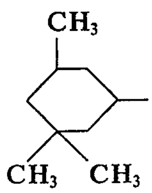

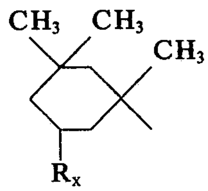

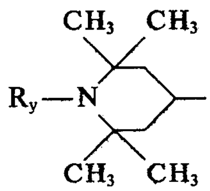

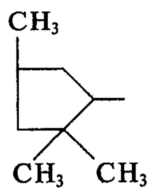

et

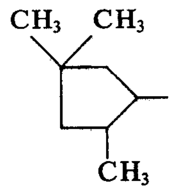

y compris les isomères de position, en particulier la forme trans avec

$R_x$ = OH, carboxyle,
$R_y$ = H, acyle,
R = hydrogène, hydroxyle, alcoxyle ou acyle, un radical alcoyle en $C_1-C_{12}$ éventuellement avec groupes hydroxyle, qui peut être à chaîne droite ou ramifiée et interrompu par des atomes d'oxygène et/ou d'azote, un radical cycloalcoyle en $C_3-C_{12}$ éventuellement avec substituants alcoyle pouvant être interrompus par des atomes d'oxygène et/ou d'azote, et/ou avec des groupes hydroxyle et/ou avec un groupe acyle aliphatique ou aromatique et/ou avec des groupes carboxyle ou carbalcoxy,
un radical alcényle ou alcinyle en $C_2-C_5$,
un radical phényle qui peut être substitué par des groupes alcoyle et/ou acyle et/ou hydroxyle et/ou dialcoylamino,
$R^1$ = hydrogène ou un radical alcoyle ou alcoxy en $C_1-C_4$ à chaîne droite ou ramifiée,
$R^2$ = hydrogène ou un radical alcoyle en $C_1-C_4$ à chaîne droite ou ramifiée éventuellement avec groupes hydroxyle, ou un radical acyle en $C_1-C_4$ et
$\gamma$ = un nombre de 0 à 1.

2. Urées avec substituants cycliques selon la revendication 1, caractérisées en ce qu'elle contiennent au moins deux substituants sur le noyau en position trans.

3. Procédé de fabrication des urées avec substituants cycliques selon les revendications 1 et 2, caractérisé en ce qu'on fait réagir les amines correspondantes avec les isocyanates ou thiocyanates ou les chlorures de carbamoyle correspondants.

4. Utilisation d'une ou plusieurs urées avec substituants cycliques selon les revendications 1 et 2 en tant qu'herbicides.

5. Utilisation d'une ou plusieurs urées avec substituants cycliques selon la revendication 1 en combinaison avec d'autres herbicides.

6. Utilisation des herbicides selon les revendications 1, 2, 4 et 5 en mélange avec des supports inertes.